# EUROPEAN PATENT SPECIFICATION

(11) **EP 3 458 229 B1**
(45) Date of publication and mention of the grant of the patent: **28.04.2021**
(21) Application number: 17800319.0
(22) Date of filing: 20.05.2017
(51) Int. Cl.: B25J 9/00, B25J 11/00, B25J 9/10, B25J 19/00

(54) **ARM SUPPORT SYSTEMS**
ARMSTÜTZSYSTEME
SYSTÈMES DE SUPPORT DE BRAS

(30) Priority: 20.05.2016 US 201662339777 P
(43) Date of publication of application: 27.03.2019
(73) Proprietor: Enhance Technologies, LLC, San Diego, CA 92121 (US)
(72) Inventor: DOYLE, Mark, C., Del Mar CA 92014 (US)
(74) Representative: Boult Wade Tennant LLP
(86) International application number: PCT/US2017/033709
(87) International publication number: WO 2017/201517

(56) References cited:
- WO-A2-2014/093408
- JP-A- 2015 164 484
- US-A1- 2006 015 214
- US-A1- 2010 204 804
- US-A1- 2015 316 204

## Description

### FIELD OF THE INVENTION:

The present invention relates to systems for supporting a user's arms, for example, to adaptive arm support systems that support one or both of a user's arms, while allowing substantially free motion, e.g., to allow the user to perform one or more tasks for extended periods of time with one or both arms extended.

### BACKGROUND:

Arm support systems may benefit from several ancillary features, which do not directly impact the support function that the arm support systems provide, but which improve the usability and safety of such systems. Disclosed herein are examples of such features.

### SUMMARY:

The present invention is directed to systems for supporting a user's arms, for example, to adaptive arm support systems or devices that support one or both of a user's arms, while allowing substantially free motion, e.g., to allow the user to perform one or more tasks for extended periods of time with one or both arms extended.

Exemplary arm support components that may be included in the embodiments herein, such as cassettes or other compensation elements, arm rest features, covers, and the like, are described in U.S. Publication Nos. 2012/ 0184880, 2014/ 0033391, 2014/ 0158839, and 2015/ 0316204, as well as in WO 2014/093408 A2. Any of the embodiments in these applications may include one or more of the additional features described herein.

The first is a feature that permits the rapid removal and attachment of a modular cassette that houses the support mechanism. Rapid removal/attachment may be beneficial when the user wishes to change the force level or force profile of the cassette, replace a defective cassette, use only one cassette, or for storage of the system.

The second feature is a protective cover that protects the user(s) from the energized mechanism in the cassette, such as springs and cables, which might fail catastrophically and otherwise exit the cassette in a dangerous fashion.

The third feature allows the user to activate or de-activate the counterbalancing effect of the cassette without disconnecting the cassette from the harness. This may be beneficial if the user is involved in activities that do not require support (such as taking a break from work), or when the device needs to be collapsed for storage.

In accordance with one embodiment, a system is provided for supporting an arm of a user that includes a harness configured to be worn on a body of a user; an arm support coupled to the harness configured to support an arm of the user; one or more compensation elements coupled to the arm support to apply an offset force to at least partially offset a gravitational force acting on the arm as the user moves and the arm support follows the movement of the user's arm, the one or more compensation elements comprising a cassette including one or more cables and springs; and a guard at least partially covering the cassette to protect the user if one or more components of the cassette fail.

In accordance with another embodiment, a system is provided for supporting an arm of a user that includes a harness configured to be worn on a body of a user; an arm support coupled to the harness configured to support an arm of the user; one or more compensation elements coupled to the arm support to apply an offset force to at least partially offset a gravitational force acting on the arm as the user moves and the arm support follows the movement of the user's arm, the one or more compensation elements comprising a cassette removably mountable to the arm support.

In accordance with still another embodiment, a system is provided for supporting an arm of a user that includes a harness configured to be worn on a body of a user; an arm support coupled to the harness configured to support an arm of the user; one or more compensation elements coupled to the arm support to apply an offset force to at least partially offset a gravitational force acting on the arm as the user moves and the arm support follows the movement of the user's arm; and a locking feature for securing the arm support when not in use.

Other aspects and features of the present invention will become apparent from consideration of the following description taken in conjunction with the accompanying drawings.

### BRIEF DESCRIPTION OF THE DRAWINGS:

It will be appreciated that the exemplary apparatus shown in the drawings are not necessarily drawn to scale, with emphasis instead being placed on illustrating the various aspects and features of the illustrated embodiments.
FIG. 1 is a rear perspective view of an exemplary embodiment of an arm support system.
FIG. 2A is a front perspective view of the arm support system of FIG. 1 with a modular arm support cassette detached from a harness of the system.
FIG. 2B is a detail of the modular cassette shown in FIG. 2A.
FIGS. 2C and 2D are side views of the modular cassette of FIG. 2A detached and attached, respectively, to the harness.
FIGS. 3A-3C are side views of the modular cassette of FIG. 2A with a cover removed to reveal internal components, showing the modular cassette in raised, horizontal, and lowered positions, respectively.
FIGS. 4A and 4B are side views of another embodiment of a modular cassette with a cover removed to reveal internal components, showing the modular cassette in raised and lowered positions, respectively.
FIGS. 5A and 5B are side views of yet another embodiment of a modular cassette in raised and lowered positions, respectively.
FIG. 5C is a perspective view of the modular cassette of FIGS. 5A and 5B with a sliding pivot cover removed.
FIGS. 6A-6C are side views of still another embodiment of a modular cassette, showing the modular cassette in raised, horizontal, and lowered positions, respectively.
FIGS. 7A and 7B are side views of another embodiment of a modular in raised and lowered positions, respectively.
FIGS. 8A and 8B are side views of yet another embodiment of a modular in raised and lowered positions, respectively.
FIGS. 9A and 9B are side views of still another embodiment of a modular cassette with a cover removed to reveal internal components, showing the modular cassette in raised and lowered positions, respectively.

### DESCRIPTION OF THE EXEMPLARY EMBODIMENTS:

In the following description, numerous details are set forth in order to provide a more thorough description of the system. It will be apparent, however, to one skilled in the art, that the disclosed system may be practiced without these specific details. In the other instances, well known features have not been described in detail so as not to unnecessarily obscure the system.

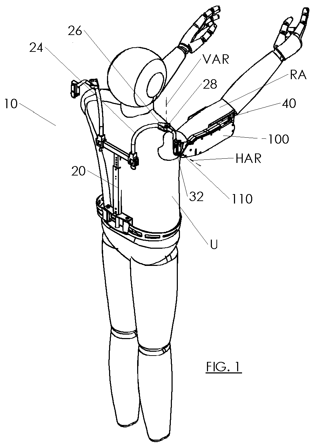

Turning to FIG. 1, a rear perspective view of an exemplary arm support system 10 is shown. A user U's right arm RA is shown supported by armrest 40, which is mounted on a cassette 100. As previously disclosed in U.S. Publication No. 2014/0158839, the cassette 100 may contain one or more springs, cables, and pulleys arranged to provide a counterbalancing lift force on the arm that varies with the position of the arm. The cassette 100 may incorporate a horizontal pivot 110, which permits counterbalancing support of the arm about a generally horizontal axis HAR. The cassette 100 is, in turn, mounted on a harness 20 at vertical pivot 28, which permits rotation of the cassette about a generally vertical axis VAR.

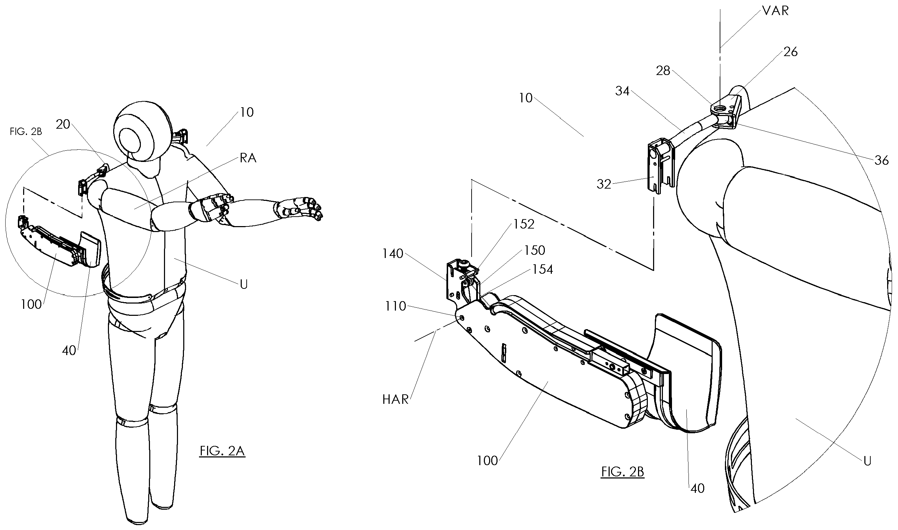

FIG. 2A is a front perspective view of the arm support system 10 showing modular cassette 100 detached from the harness 20. The cassette 100 may be rapidly attached to (or detached from) the harness 20 as desired to permit rapid changing of the cassette 100, for example, to change the lift force level or to change the lift profile in response to the requirements of the user or the task, to replace a damaged cassette, or for storage. For example, in one system, a plurality of cassettes (not shown) may be provided that have different force profiles, and the user may select and attach a desired cassette from the set, e.g., based on a desired force profile. For example, in some applications, different cassettes may apply different supporting forces when the arm RA is extended and/or raised, e.g., such that a user may select a higher supporting force profile cassette when the user anticipates lifting or carrying heavy objects, e.g., heavy tools (not shown), or spending longer time periods with the arm extended, while in other applications, a user may select a lower force cassette if the user wants less support during use.

FIG. 2B is a detail perspective view of the arm support system 10 of FIG. 2A. The cassette 100 may be rapidly attached to (or detached from) the harness 20 by inserting hub 140 on the cassette 100 into a socket 32 in the harness 20. Hub 140 and socket 32 may contain cooperating locking features (as described below) to keep the cassette 100 attached to the harness 20. For example, as shown, the harness 20 may include a harness bracket 26 and a shoulder bracket 34 having a first end 36 pivotally coupled to the harness bracket 26 at a vertical pivot 28 such that the socket 32 on a second end of the shoulder bracket 34 pivots substantially horizontally relative to the harness 20. The socket 32 may be pivotally coupled to the shoulder bracket 26 such that the socket 32 may pivot about a horizontal pivot, e.g., to allow the cassette 100 to rotate substantially vertically as the user raises and lowers the right arm RA

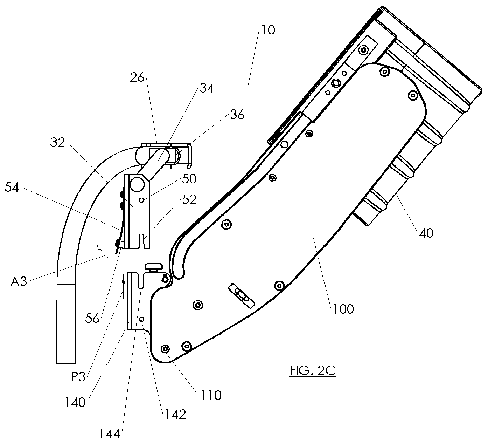

FIG. 2C is a detail side view of the cassette/harness interface shown in FIG. 2B. As cassette lock spring 54 is pulled away from the socket 32 (by the user), approximately along arc A3, lock post 56 is pulled out of the way of cassette 100. The cassette 100 is pushed up into the socket 32 approximately along path P3. The hub 140 on the cassette 100 fits into socket 32. As the hub 140 of the cassette 100 enters the socket 32, notch 144 in the hub 140 receives pin 50 in the socket 32, as the notch 52 in the socket 32 receives pin 142 in the hub 140.

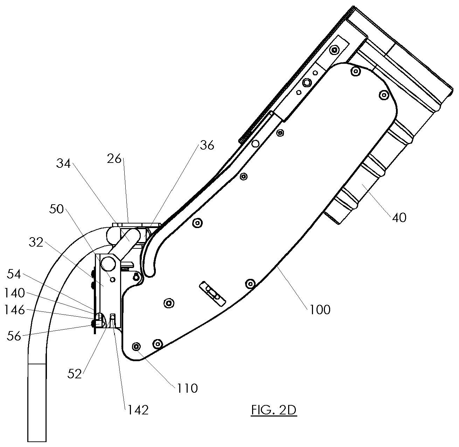

FIG. 2D is a detail side view of the cassette/harness interface shown in FIG. 2C, showing the cassette 100 locked into the socket 32. The notch 144 in the hub 140 holds the pin 50 in the socket 32, and the notch 52 in the socket 32 holds the pin 142 in the hub 140. Cassette lock spring 54 is un-deflected, and lock post 56 is received in the notch 146 in the hub 140, thus locking the cassette 100 into the socket 32.

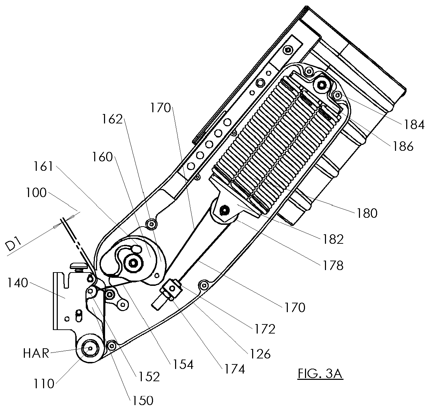

FIG. 3A is a side view of the cassette 100 of arm support system 10 with one side cover removed to show exemplary components of the cassette 100. Anchor block 150 is latched to the hub 140 during normal operation, e.g., by features disclosed below. Chassis 126 is pivotally mounted to the hub 140 at pivot 110, and rotates about horizontal axis HAR. Chassis 126 is shown in the raised position, approximately fifty degrees (50°) above horizontal, consistent with a user working with their arm raised. First cable 154 is pivotally attached to the anchor block 150 at attachment point 152, and attached to first pulley 160 at first cable attachment point 161. Second cable 170 is attached to second pulley 162 at second cable attachment point (not shown). First and second pulleys 160, 162 are joined together and rotate as one. Optionally, the second cable 170 may wrap around an optional reversing pulley 178, and is attached to the chassis 126 at adjustable anchor 172. One or more springs 180, which may vary in number and rate, are attached to the reversing pulley 178 by frame 182 and to the chassis 126 at pivot 184 by rear frame 186. As shown, the cassette chassis 126 is in close proximity to the hub 140, as depicted by dimension D1.

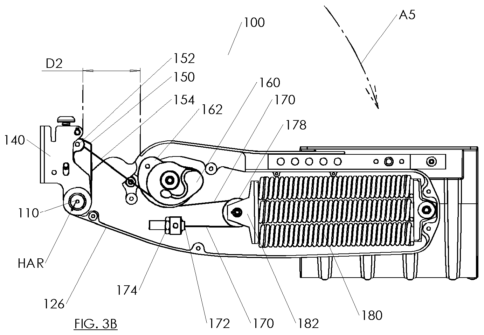

FIG. 3B shows the cassette of FIG. 3A declined to an approximately horizontal position (consistent, for example, with a user lowering their arm to a horizontal position) approximately along arc A5. First and second pulleys have together rotated clockwise as first cable 154 extends and un-wraps from first pulley 160 in response to the rotation of chassis 126. The second cable 170 is simultaneously taken up on the second pulley 162, thereby stretching the springs 180. As the springs 180 extend, the force in in them increases, increasing the tension force in both cables 154, 170. As shown, the top edge of the cassette chassis 126 has moved away from the hub 140, as depicted by dimension D2, opening a gap between the chassis 126 and the hub 140.

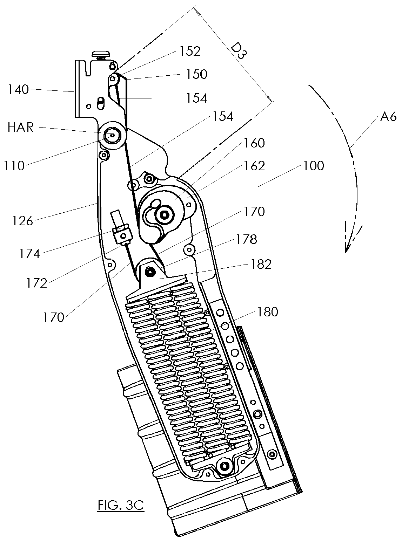

FIG. 3C shows the cassette of FIG. 3B declined to approximately seventy degrees (70°) below horizontal (consistent, for example, with the user lowering their arm to work near waist level) approximately along arc A6. The first and second pulleys 160, 162 have together rotated further clockwise as first cable 154 extends and un-wraps from the first pulley 160 in response to the rotation of the chassis 126. The second cable 170 is simultaneously further taken up on the second pulley 162, thereby further stretching the springs 180, and further increasing the tension force in both cables 154, 170. As shown, the top edge of the cassette chassis 126 has moved further away from the hub 140, as depicted by dimension D3, opening a large gap between the chassis 126 and hub 140.

As the tension in the cables increases, the danger to the user of a catastrophic cable failure increases. For example, a cable failure that occurs with the cassette 100 in the position shown in FIG. 3C, with dangerous tension in the cable 154, could result in the cable exiting the gap between the cassette and hub at high speed, which might endanger the user or others.

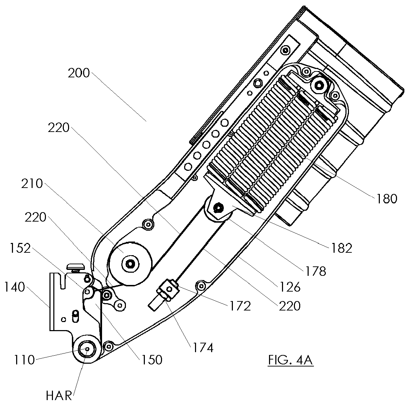

FIG. 4A is a side view of an alternative single cable cassette 200 of arm support system 10 with one side cover removed to show internal components. As in FIGS. 3A-3C, anchor block 150 is latched to hub 140 during normal operation, by features disclosed below. Chassis 126 is pivotally mounted to the hub 140 at pivot 110, and rotates about horizontal axis HAR. The chassis 126 is shown in a raised position, approximately fifty degrees (50°) above horizontal, consistent with the user working with their arm raised. Single cable 220 is pivotally attached to anchor block 150 at attachment point 152. The single cable 220 wraps around a portion of main pulley 210, around optional reversing pulley 178, and is attached to the chassis 126 at adjustable anchor 172. One or more springs 180, which may vary in number and rate, are attached to the reversing pulley 178 by frame 182 and to the chassis 126 at pivot 184 by rear frame 186.

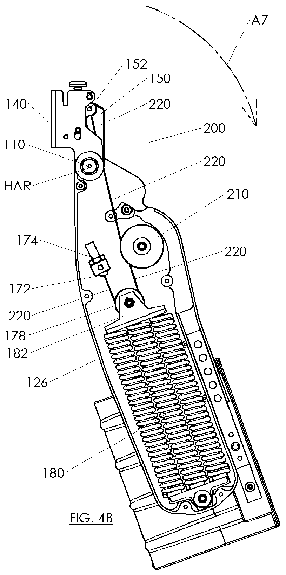

FIG. 4B is a side view of the cable cassette 200 of arm support system 10 of FIG. 4A with the cassette 200 declined to approximately seventy degrees (70°) below horizontal (consistent, for example, with the user lowering their arm to work near waist level) approximately along arc A7. As the cassette 200 is declined, the single cable 220 needs to extend in response. Being un-extendable, the cable 220 applies a tension force on the reversing pulley 178, thereby stretching the springs 180, and increasing the tension force in the cable 220. As the tension in the cable 220 increases, the danger to the user of a catastrophic cable failure increases. For example, a cable failure that occurs with the cassette 200 in the position shown in FIG. 4A, with dangerous tension in the cable 220, could result in the cable 220 or other components exiting the cassette 200 at high speed, which might endanger the user or others.

Thus, there is a need for a cover system for an arm support system cassette, to protect users and others from possible cable or other mechanical failure.

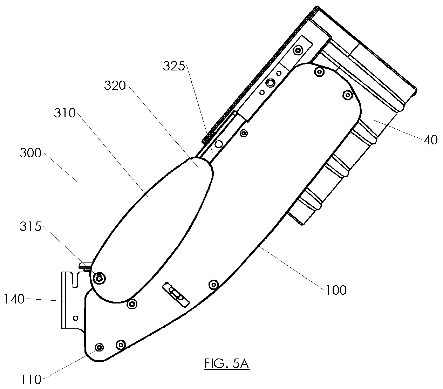

FIG. 5A is a side view of an exemplary embodiment of a protected cassette 300 for an arm support system 10 (such as that shown in FIG. 1) with a sliding safety cover 310 installed on cassette 100. Protected cassette 300 is shown in the raised position, approximately fifty degrees (50°) above horizontal, consistent with the user working with their arm raised. The sliding cover 310 is pivotally attached to hub 140 at cover pivot 315. Cover front end 320 is captured in track 325, along which it can slide as required. The sliding cover 310 may be rigid or semi rigid, or made of rigid and semi-rigid components.

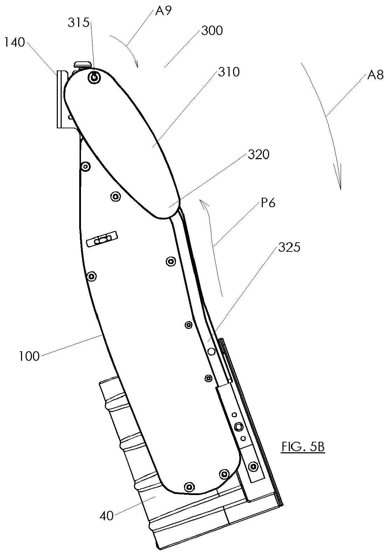

FIG. 5B is a side view of the protected cassette 300 of FIG. 5A shown with the cassette 300 declined to approximately seventy degrees (70°) below horizontal (consistent, for example with the user lowering their arm to work near waist level) approximately along arc A8. In response to the motion of the cassette 300, the sliding cover 310 has pivoted about the cover pivot 315 approximately along arc A9, and the cover front end 320 has travelled along the track 325, approximately along path P6.

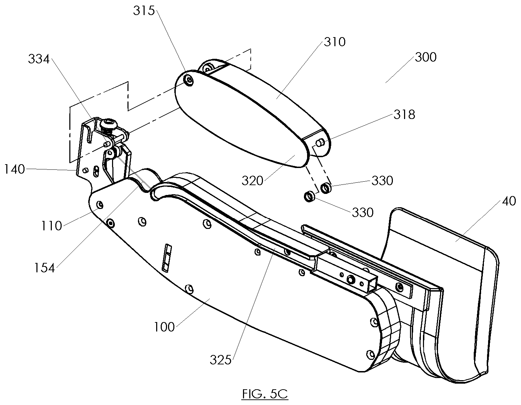

FIG. 5C is a perspective view of the protected cassette 300 of FIGS. 5A and 5B, provided to clarify the sliding action. The sliding cover pivot 315 pivotally attaches to the hub 140 at sliding cover shaft 334. The inside of the cover front end 320 includes track posts 318 (only one shown), which are captured within (and travel along) the track 325. Optional post rollers 330 may be provided to ease the sliding motion.

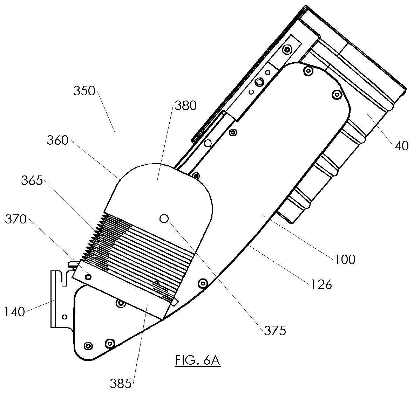

FIG. 6A is a side view of another embodiment of a protected cassette 350 for an arm support system 10 with an expanding safety cover 360 installed on cassette 100. Protected cassette 350 is shown in the raised position, approximately fifty degrees (50°) above horizontal, consistent with the user working with their arm raised. Expanding cover 360 is optionally pivotally attached to hub 140 at cover pivot 370. Cover front end 320 may be fixed to the cassette 100 at attachment point 375. In this cassette position, (raised) pleats 365 may be relatively compressed. The expanding cover 360 may be flexible, semi-rigid, made from materials used in bellows manufacturing such as rubber or fabric, and may contain rigid or semi-rigid elements to help maintain proper shape.

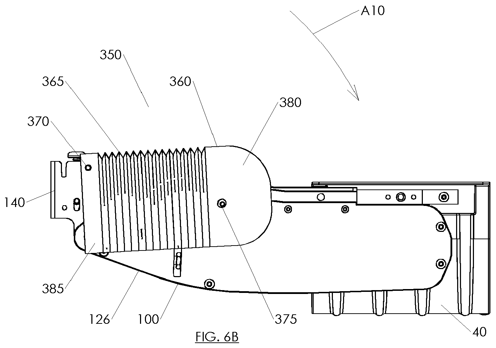

FIG. 6B is a side view of the protected cassette 350 of FIG. 6A with the cassette 100 declined to approximately horizontal (consistent, for example, with the user lowering their arm to work near shoulder level) approximately along arc A10. As the distance between the hub 140 and cassette chassis 126 increases, the pleats 365 expand to accommodate the change in length.

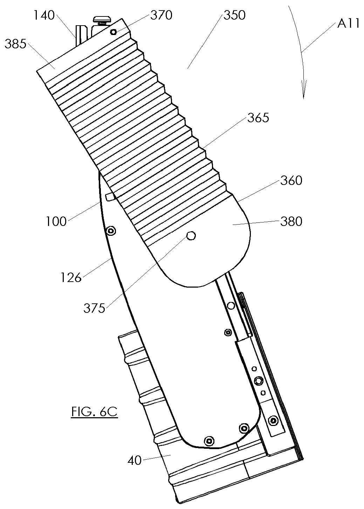

FIG. 6C is a side view of the protected cassette 350 of FIG. 6B with the cassette 100 declined to approximately seventy degrees (70°) below horizontal (consistent, for example, with the user lowering their arm to work near waist level) approximately along arc A11. As the distance between the hub 140 and cassette chassis 126 further increases, the pleats 365 expand further to accommodate the change in length.

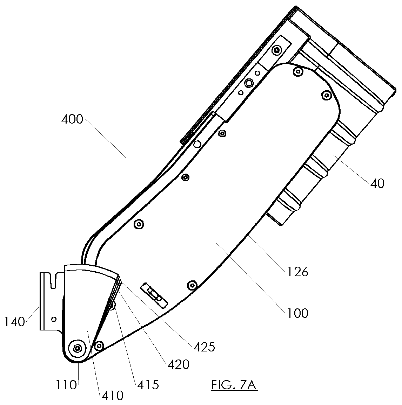

FIG. 7A is a side view of yet another embodiment of a protected cassette 400 for an arm support system 10 with a nested segment safety cover 410-425 installed on cassette 100. The protected cassette 400 is shown in the raised position, approximately fifty degrees (50°) above horizontal, consistent with the user working with their arm raised. Nested segment safety cover 410-425 is optionally pivotally attached to hub 140 at pivot 110. The nested segment 410 may be fixed to the hub 140, and nested segment 425 may be fixed to chassis 126. Nested segments 410-425 may be sized to fit within each other. For example, segment 415 fits inside of segment 410, segment 420 fits inside segment 415, etc. In this cassette position (raised) nested segments 410, 415, 420, and 425 may be nested within each other. Segments 410-425 may be rigid, semi-rigid, or made of rigid and semi-rigid components.

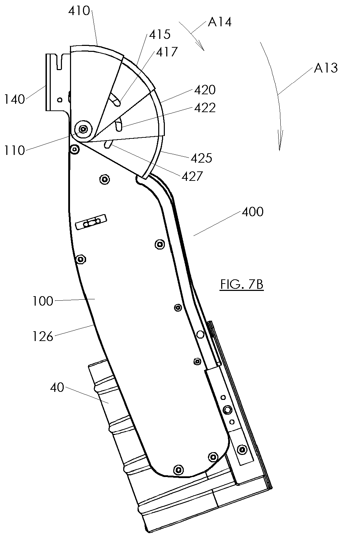

FIG. 7B is a side view of the protected cassette 400 of FIG. 7A with the cassette declined to approximately seventy degrees (70°) below horizontal (consistent, for example, with the user lowering their arm to work near waist level) approximately along arc A13. The nested segments 410, 415, 420, and 425 are show spread apart, approximately along arc A14, in response to the motion of the chassis 126. Features that keep the nested segments 410-425 in the desired relationship are contemplated. For example, slots and posts may be added to allow a given segment to pull an adjacent segment along with it, and to prevent the segments from separating entirely, but still permitting them to nest properly. For example, slot 417 in segment 415 cooperates with a post in segment 420 (post not shown) to allow segment 420 to pull segment 415 out of segment 410. Slot 422 in segment 420 cooperates with a post in segment 425 (post not shown) to allow segment 425 to pull segment 420 out of segment 415. Slot 427 in segment 425 cooperates with a post in segment 425 (post not shown) to allow segment 425 to pull segment 420 out of segment 415.

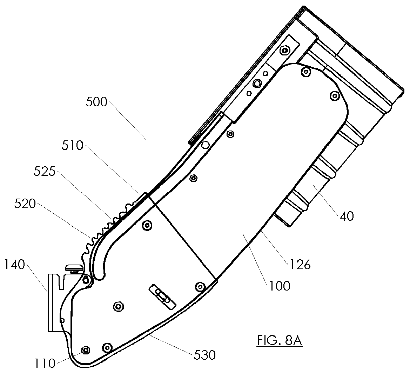

FIG. 8A is a side view of another protected cassette 500 for an arm support system 10 with a flexible protective cover 510 (shown as translucent for clarity). Flexible cover 510 is attached to hub 140 and cassette chassis 126. Protected cassette 500 is shown in the raised position, approximately fifty degrees (50°) above horizontal, consistent with the user working with their arm raised. As shown, flexible cover top surface 520 may be compressed or bunched-up into one or more folds 525 of excess material, to accommodate motion of the cassette 100, while flexible cover lower surface 530 may be relatively taut. The flexible cover 510 may be made from elastic or non-elastic fabric or film, may be solid, woven, or mesh, and may contain plates, wires, rods, cables, or other components to increase durability, increase safety, and control shape. If made from elastic materials, flexible cover top surface may not need to be compressed or bunched-up into one or more folds 525 of excess material to accommodate motion of the cassette 100.

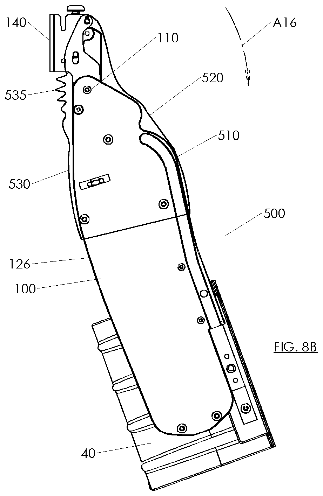

FIG. 8B is a side view of protected cassette 500 of FIG. 8A, shown declined to approximately seventy degrees (70°) below horizontal (consistent, for example with the user lowering their arm to work near waist level) approximately along arc A16. As shown, the flexible cover top surface 520 is now relatively taut, and the flexible cover lower surface 530 may now be compressed or bunched-up into one or more folds 535, in response to the motion of the chassis 126. If made from elastic materials flexible cover lower surface 530 may not need to be compressed or bunched-up into one or more folds 535 of excess material to accommodate motion of the cassette.

Other safety devices or covers are contemplated. For example, a cover in the form of a coil of relatively flat strip material (not shown) that can coil and uncoil in response to the motion of the cassette can be used to cover the mechanism inside of the cassette. The coil might be mounted on the hub 140, on a spring-loaded drum (as in a roller-shade for a window), and the other end of the strip might be attached to the cassette. In response to the motion of the cassette the coil is able to rotate, as the strip is pulled off of the coil, and thus the strip is pulled out and extended over the mechanism in the cassette. The spring in the drum acts to pull the strip back onto the drum as necessary. Another example is to provide one or more loops of wire or cord over the cassette that can serve to intercept the cable if it breaks.

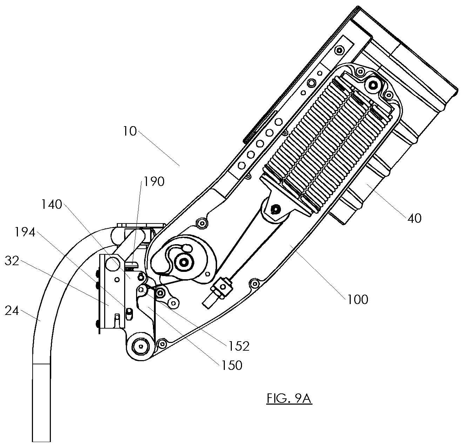

FIG. 9A is a detailed side view of FIG. 2D, provided to illustrate a feature of cassette 100 used for activation and deactivation of the cassette 100. In some cases, it is desirable to deactivate the lift function of the cassette 100, while keeping the cassette 100 attached to the harness. Examples include when the user does not want or need the counterbalancing lift force, or when the user is storing the device when not in use. Cassette release button 190, maintained in a first position by a spring, is connected to lock pin 194. In use, lock pin 194 holds anchor block 150 in position against hub 140, as shown in FIGS. 3A-3C.

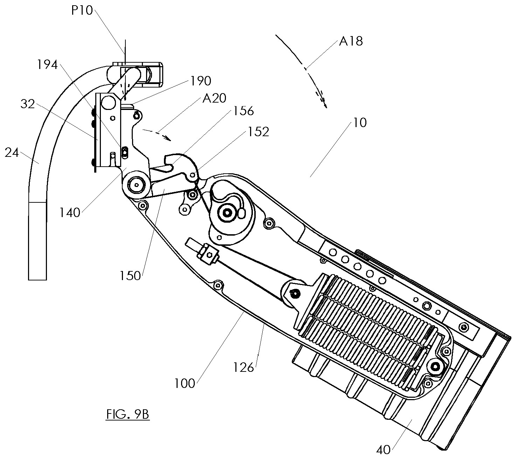

FIG. 9B is a detailed view of the cassette 100 of FIG. 9A, showing the cassette deactivated. To deactivate the cassette 100, the user depresses cassette release button 190, approximately along path P10. This action pushes lock pin 194 downward and out of engagement with hook feature 156 in anchor block 150, thereby releasing anchor block 150 from hub 140. Once released, the anchor block 150, along with cassette chassis 126 and all components attached thereto, are able to move freely, without the counterbalancing force described above (for example, along arc A18). When the user wishes to re-activate the cassette 100, the cassette 100 can be lifted up to snap the lock pin 194 back into the hook feature 156 in the anchor block 150.

While the cassette-harness detachment, safety cover, and cassette deactivation features disclosed above are described in the context of arm support devices based on cables, springs, and pulleys, they are all also applicable to systems that are not cable or spring based. They may also be used in powered arm support device, and, as applicable, in systems designed to support other portions of the body, such as the legs.

It will be appreciated that elements or components shown with any embodiment herein are exemplary for the specific embodiment and may be used on or in combination with other embodiments disclosed herein.

While the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents and alternatives falling within the scope of the appended claims.

## Claims

1. A system (10) for supporting an arm (RA) of a user (U), comprising:
a harness (20) configured to be worn on a body of a user;
an arm support coupled to the harness configured to support an arm of the user;
one or more compensation elements coupled to the arm support to apply an offset force to at least partially offset a gravitational force acting on the arm as the user moves and the arm support follows the movement of the user's arm, the one or more compensation elements comprising a cassette (100, 200) including one or more cables (154, 170) and springs (180); and
a guard (310, 360, 410, 510) at least partially covering the cassette to protect the user if one or more components of the cassette fail.

2. The system of claim 1, wherein the arm support comprises a shoulder bracket (34) pivotally coupled to the harness such that the shoulder bracket rotates horizontally about a vertical axis (28), and wherein the cassette is coupled the shoulder bracket such that the cassette pivots vertically about a horizontal axis (HAR) relative the shoulder bracket, the cassette configured to apply an offset force to at least partially offset a gravitational force acting on the arm as the user lowers and raises the arm and the arm support follows the movement of the user's arm.

3. The system of claim 1 or 2, wherein the guard comprises an expandable cover (360) including a first end (370) coupled to the shoulder bracket and a second end (375) coupled to the cassette.

4. The system of claim 1 or 2, wherein the guard (310, 360, 410, 510) is movable relative to the cassette as the arm support moves between one or more positions.

5. The system of claim 1 or 2, wherein the guard (360, 410, 510) is expandable and contractible as the arm support moves between one or more positions.

6. The system of claim 1 or 2, wherein the guard comprises an expandable cover (360, 410, 510) including a first end coupled to the harness and a second end coupled to the cassette.

7. The system of claim 1 or 2, wherein the guard comprises a pleated cover (360, 510) including a plurality of expandable pleats (365, 525) to accommodate expansion and contraction of the cover as the arm support moves.

8. The system of claim 1 or 2, wherein the guard comprises an expandable cover (410) including a plurality of segments (415, 420, 425) that nest relative to one another to accommodate expansion and contraction of the cover as the arm support moves.

9. The system of claim 1 or 2, wherein the guard comprises a slidable cover (310) mounted to the cassette.

10. The system of any preceding claim, wherein the cassette is removably mountable to the arm support.

11. The system of claim 2, , wherein the arm support comprises a connector (32) pivotally coupled to a second end of the shoulder bracket (34) such that the connector rotates vertically about a horizontal axis, and wherein the cassette includes a first end including a mating connector (140) connectable to the connector to mount the cassette to the second end of the shoulder bracket.

12. The system of claim 11, wherein the connector comprises a socket and the mating connector comprises a hub connectable to the socket, the connector further comprising a lock (56) for releasably locking the socket and hub together.

13. The system of claim 12, further comprising a locking feature for disengaging the cassette when not in use.

14. The system of claim 13, wherein the locking feature comprises a locking pin (194) on the cassette that is engaged with the socket (156) when the cassette is in use, and a release actuator (190) that disengages the locking pin from the socket, thereby causing the cassette to be released to pivot relative to the socket to an inactive position.

## Patentansprüche

1. System (10) zum Stützen eines Arms (RA) eines Benutzers (U), umfassend:
ein Gurtzeug (20), das zum Tragen an einem Körper eines Benutzers konfiguriert ist;
eine Armstütze, die mit dem Gurtzeug gekoppelt ist und zum Stützen eines Arms des Benutzers konfiguriert ist;
ein oder mehrere Kompensationselemente, die mit der Armstütze gekoppelt sind, um eine Kompensationskraft aufzubringen, um eine Gravitationskraft, die auf den Arm wirkt, zumindest teilweise auszugleichen, wenn sich der Benutzer bewegt und die Armstütze der Bewegung des Arms des Benutzers folgt, wobei die ein oder mehreren Kompensationselemente eine Kassette (100, 200) umfassen, die ein oder mehrere Seile (154, 170) und Federn (180) enthält; und
eine Schutzvorrichtung (310, 360, 410, 510), der die Kassette zumindest teilweise abdeckt, um den Benutzer zu schützen, wenn eine oder mehrere Komponenten der Kassette versagen.

2. System nach Anspruch 1, bei dem die Armstütze eine Schulterhalterung (34) umfasst, die schwenkbar mit dem Gurtzeug gekoppelt ist, so dass sich die Schulterhalterung horizontal um eine vertikale Achse (28) dreht, und bei dem die Kassette mit der Schulterhalterung gekoppelt ist, so dass die Kassette vertikal um eine horizontale Achse (HAR) relativ zur Schulterhalterung schwenkt, wobei die Kassette konfiguriert ist, um eine Ausgleichskraft auszuüben, um eine Schwerkraft zumindest teilweise auszugleichen, die auf den Arm wirkt, wenn der Benutzer den Arm senkt und hebt und die Armstütze der Bewegung des Arms des Benutzers folgt.

3. System nach Anspruch 1 oder 2, bei dem die Schutzvorrichtung eine dehnbare Abdeckung (360) umfasst, die ein mit der Schulterhalterung verbundenes erstes Ende (370) und ein mit der Kassette verbundenes zweites Ende (375) aufweist.

4. System nach Anspruch 1 oder 2, bei dem die Schutzvorrichtung (310, 360, 410, 510) relativ zur Kassette beweglich ist, wenn sich die Armstütze zwischen ein oder mehreren Positionen bewegt.

5. System nach Anspruch 1 oder 2, bei dem die Schutzvorrichtung (360, 410, 510) dehnbar und zusammenziehbar ist, wenn sich die Armstütze zwischen ein oder mehreren Positionen bewegt.

6. System nach Anspruch 1 oder 2, bei dem die Schutzvorrichtung eine dehnbare Abdeckung (360, 410, 510) umfasst, die ein erstes Ende, das mit dem Gurtzeug verbunden ist, und ein zweites Ende, das mit der Kassette verbunden ist, aufweist.

7. System nach Anspruch 1 oder 2, bei dem die Schutzvorrichtung eine gefaltete Abdeckung (360, 510) umfasst, die eine Mehrzahl von dehnbaren Falten (365, 525) enthält, um die Dehnung und Zusammenziehung der Abdeckung aufzunehmen, wenn sich die Armstütze bewegt.

8. System nach Anspruch 1 oder 2, bei dem die Schutzvorrichtung eine dehnbare Abdeckung (410) umfasst, die eine Mehrzahl von Segmenten (415, 420, 425) enthält, die sich relativ zueinander verschachteln, um eine Dehnung und Zusammenziehung zu ermöglichen, wenn sich die Armstütze bewegt.

9. System nach Anspruch 1 oder 2, bei dem die Schutzvorrichtung eine an der Kassette befestigte verschiebbare Abdeckung (310) umfasst.

10. System nach einem der vorhergehenden Ansprüche, bei dem die Kassette abnehmbar an der Armstütze befestigt ist.

11. System nach Anspruch 2, bei dem die Armstütze einen Verbinder (32) umfasst, der schwenkbar mit einem zweiten Ende der Schulterhalterung (34) gekoppelt ist, so dass sich der Verbinder vertikal um eine horizontale Achse dreht, und die Kassette ein erstes Ende mit einem Gegenverbinder (140) umfasst, der mit dem Verbinder verbindbar ist, um die Kassette an dem zweiten Ende der Schulterhalterung zu befestigen.

12. System nach Anspruch 11, bei dem der Verbinder eine Buchse umfasst und der Gegenverbinder eine Nabe umfasst, die mit der Buchse verbindbar ist, wobei der Verbinder ferner eine Verriegelung (56) zum lösbaren Verriegeln der Buchse und der Nabe aneinander umfasst.

13. System nach Anspruch 12, das ferner ein Verriegelungsmerkmal zum Lösen der Kassette bei Nichtgebrauch umfasst.

14. System nach Anspruch 13, bei dem das Verriegelungsmerkmal einen Verriegelungsstift (194) an der Kassette, der mit der Buchse (156) in Eingriff steht, wenn die Kassette in Gebrauch ist, und einen Freigabeaktuator (190) umfasst, der den Verriegelungsstift von der Buchse löst und dadurch die freizugebende Kassette veranlasst, relativ zur Buchse in eine inaktive Position zu schwenken.

## Revendications

1. Système (10) pour supporter un bras (RA) d'un utilisateur (U), comprenant :
un harnais (20) configuré pour être porté sur le corps d'un utilisateur ;
un support de bras couplé au harnais configuré pour supporter un bras de l'utilisateur ;
un ou plusieurs éléments de compensation couplés au support de bras pour appliquer une force de compensation pour compenser au moins partiellement une force de gravitation agissant sur le bras lorsque l'utilisateur se déplace et le support de bras suit le mouvement du bras de l'utilisateur, les un ou plusieurs éléments de compensation comprenant une cassette (100, 200) comportant un ou plusieurs câbles (154 , 170) et ressorts (180) ; et
un protecteur (310, 360, 410, 510) recouvrant au moins partiellement la cassette pour protéger l'utilisateur en cas de défaillance d'un ou plusieurs composants de la cassette.

2. Système de la revendication 1, dans lequel le support de bras comprend un support d'épaule (34) couplée en pivotement au harnais de sorte que le support d'épaule tourne horizontalement autour d'un axe vertical (28), et où la cassette est couplée à le support d'épaule de sorte que la cassette pivote verticalement autour d'un axe horizontal (HAR) par rapport au support d'épaule, la cassette étant configurée pour appliquer une force de compensation pour compenser au moins partiellement une force de gravitation agissant sur le bras lorsque l'utilisateur abaisse et lève le bras et le support de bras suit le mouvement du bras de l'utilisateur.

3. Système de la revendication 1 ou 2, dans lequel le protecteur comprend un couvercle extensible (360) comportant une première extrémité (370) couplée au support d'épaule et une deuxième extrémité (375) couplée à la cassette.

4. Système de la revendication 1 ou 2, dans lequel le protecteur (310, 360, 410, 510) est mobile par rapport à la cassette lorsque le support de bras se déplace entre une ou plusieurs positions.

5. Système de la revendication 1 ou 2, dans lequel le protecteur (360, 410, 510) est extensible et rétractable lorsque le support de bras se déplace entre une ou plusieurs positions.

6. Système de la revendication 1 ou 2, dans lequel le protecteur comprend un couvercle extensible (360, 410, 510) comportant une première extrémité couplée au harnais et une deuxième extrémité couplée à la cassette.

7. Système de la revendication 1 ou 2, dans lequel le protecteur comprend un couvercle plissé (360, 510) comportant une pluralité de plis extensibles (365, 525) pour permettre l'extension et la rétraction du couvercle lorsque le support de bras se déplace.

8. Système de la revendication 1 ou 2, dans lequel le protecteur comprend un couvercle extensible (410) comportant une pluralité de segments (415, 420, 425) qui s'emboîtent les uns par rapport aux autres pour permettre l'extension et la rétraction du couvercle lorsque le support de bras se déplace.

9. Système de la revendication 1 ou 2, dans lequel le protecteur comprend un couvercle coulissant (310) monté sur la cassette.

10. Système de l'une des revendications précédentes, dans lequel la cassette peut être montée de manière amovible sur le support de bras.

11. Système de la revendication 2, dans lequel le support de bras comprend un connecteur (32) couplé en pivotement à une deuxième extrémité du support d'épaule (34) de sorte que le connecteur tourne verticalement autour d'un axe horizontal, et où la cassette comporte une première extrémité comportant un connecteur homologue (140) pouvant être relié au connecteur pour monter la cassette sur la deuxième extrémité du support d'épaule.

12. Système de la revendication 11, dans lequel le connecteur comprend un embout femelle et le connecteur homologue comprend un moyeu pouvant être relié à l'embout femelle, le connecteur comprenant en outre un verrou (56) pour verrouiller de manière amovible l'embout femelle et le moyeu ensemble.

13. Système de la revendication 12, comprenant en outre un élément de verrouillage pour désengager la cassette lorsqu'elle n'est pas utilisée.

14. Système de la revendication 13, dans lequel l'élément de verrouillage comprend une broche de verrouillage (194) sur la cassette qui est engagée avec l'embout femelle (156) lorsque la cassette est en cours d'utilisation, et un actionneur de libération (190) qui désengage la broche de verrouillage de l'embout femelle, amenant ainsi la cassette à être libérée pour pivoter par rapport à l'embout femelle vers une position inactive.
